# EUROPEAN PATENT SPECIFICATION

(11) **EP 3 576 708 B1**
(45) Date of publication and mention of the grant of the patent: **12.07.2023**
(21) Application number: 18715952.0
(22) Date of filing: 24.01.2018
(51) Int. Cl.: A61H 33/00, A61H 33/14, A61H 35/00

(54) **APPARATUS FOR TRANSCUTANEOUS TREATMENT BY GAS**
VORRICHTUNG ZUR TRANSKUTANEN BEHANDLUNG DURCH GAS
APPAREIL DE TRAITEMENT TRANSCUTANÉ PAR GAZ

(30) Priority: 31.01.2017 HU 1700043; 02.08.2017 HU 1700334
(43) Date of publication of application: 11.12.2019
(73) Proprietor: Berta, Zsolt Zoltán, 3078 Bátonyterenye (HU); Tóth, Béla Ernõ, 1037 Budapest (HU)
(72) Inventor: Berta, Zsolt Zoltán, 3078 Bátonyterenye (HU); Tóth, Béla Ernõ, 1037 Budapest (HU)
(74) Representative: Danubia Patent & Law Office LLC
(86) International application number: PCT/HU2018/050005
(87) International publication number: WO 2018/142171

(56) References cited:
- WO-A1-2004/047710
- US-A1- 2005 056 279
- US-A1- 2011 125 083

## Description

The present invention relates to a treatment apparatus, in particular for transcutaneous therapeutic or preventive treatment by gaseous materials or gas mixtures.

The favourable effects of natural gases or gas mixtures, for example carbon dioxide, for treating various health conditions or diseases have been known for thousands of years. The appropriately selected gases take their effects in a transcutaneous way, i.e. by adsorption through the skin. The development of devices capable of feeding gases or gas mixtures into a treatment space made it feasible to perform human applications and therapeutic treatments under artificial circumstances. In addition to the natural gases, gas mixtures or vapour mixtures, one can also use synthetic gas mixtures, medical gases or gas compounds comprising components in portions different from that of the ambient air. Depending on the components of the gases or gas mixtures, the indications also vary. For example, the carbon dioxide gas has shown favourable results in the treatment of angiologic diseases.

With respect to the fact that some of the components of the gas mixtures or vapour mixtures used in the treatments, when inbreathed, have harmful or toxic effects, the performance of the treatment in a safe and controlled way is of great importance. At the design of devices using such gas mixtures or vapour mixtures, a safety system composed of cooperating and integrated elements should be involved, which can provide a successful and secure therapeutic treatment for the patient subject to the treatment, the persons around and also for the operating staff.

The documents US 2013/079703 and US 2011/125083 disclose a carbon dioxide treatment device wherein the body of the patient is accommodated in a hermetically sealed inner space of the device, while his head is kept outside the device, in the open air. Within the inner space of the device there is a pressurized gas, the desired pressure of which is maintained by means of a passive discharge valve.

The documents US 2005/056279 and WO 2004/047710 also disclose gas treatment devices which comprise a hermetically sealed cabinet with a ventilation unit for evacuating gas from the cabinet.

A drawback of the above devices is that in case of emergency, the active and substantially quick discharge of the gas from the inner space of the cabinet into another space hermetically separated from the treatment space, for example into the open air, has not been solved.

It is an object of the present invention to provide a treatment apparatus that is adapted for an immediate and fast discharge of the treating gas from the inner space of the cabinet into another hermetically separated space at the end of the treatment using gaseous materials or even at an emergency situation occurring in course of a treatment.

The invention is based on the inventive idea that if the treatment apparatus is provided with a ventilation unit that at a normal treatment or at an emergency stop, conveys the gas from the treatment cabinet within a short time into a space hermetically separated from the room of treatment, e.g. into the open air, then the treatment can be performed with the highest safety in an indoor treating room even with using gases that are otherwise harmful for the human health.

The above objects are achieved by providing an apparatus according to claim 1.

Preferably, the cabinet further comprises at least one sensor for indicating opening of an openable panel, said sensor being coupled to the control unit. As an openable panel, the cabinet further comprises a door on its side, preferably on its front side, and in this case the at least one opening indication sensor is a sensor adapted to detect opening of the door.

As an additional openable panel, the apparatus may further comprise an openable or removable lid on the upper side of the cabinet, and in this case, a sensor may be associated with the lid for indicating displacement of the lid.

Preferably, the apparatus further comprises a displacement sensor built in a back rest of the seat or in an inner side wall of the cabinet, said displacement sensor being in the form of a proximity sensor or a pressure sensor.

A preferred embodiment of the apparatus further comprises a sensor for measuring the gas concentration around the cabinet and for indicating that the surrounding gas concentration reaches a preset concentration, said sensor being coupled to the control unit.

It is also preferred that the apparatus further comprises at least one emergency stop switch coupled to the control unit.

The invention will now be described in detail with reference to the drawings. In the drawings:
Figure 1 is a functional block diagram of a preferred embodiment of the apparatus according to the invention, in which a cabinet is shown in a sectional view.

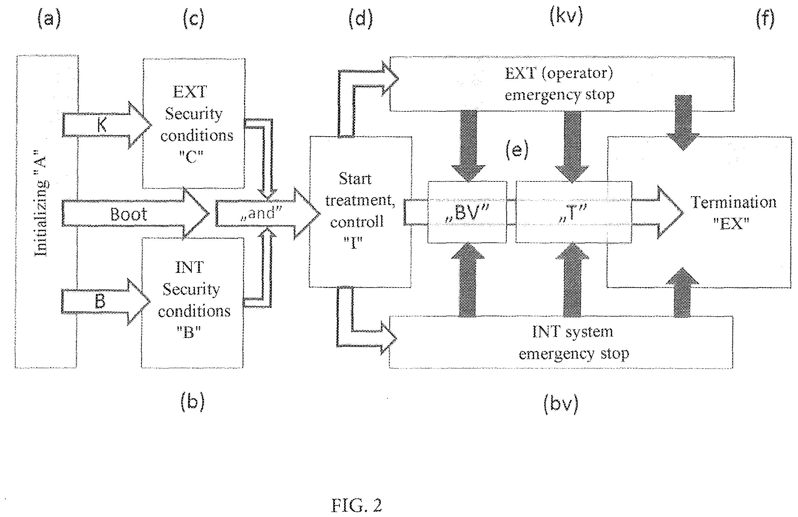

Figure 2 is a flow diagram illustrating the operation of a computer program controlling the safety functions of the apparatus according to the invention.

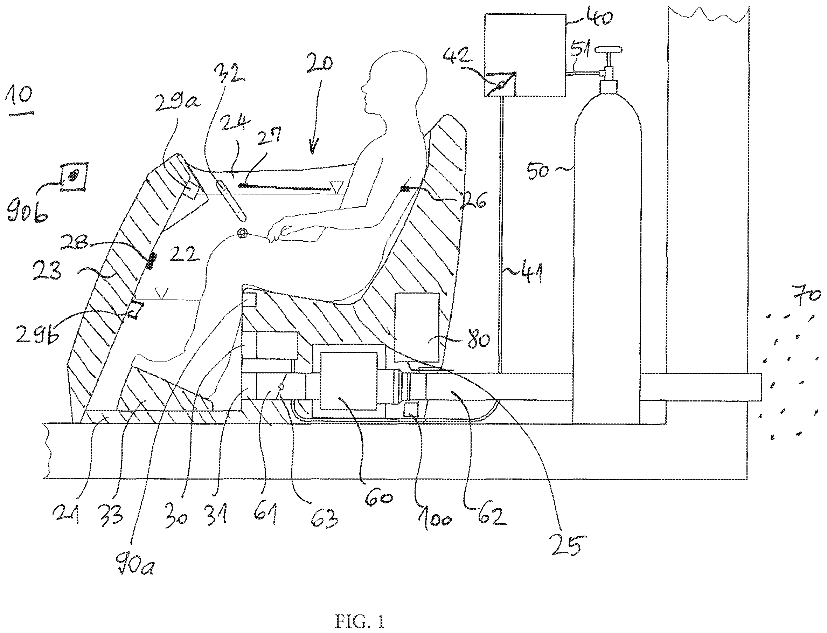

The design of the apparatus 10 according to the present invention is shown in Figure 1. The apparatus 10 comprises a cabinet 20, preferably having rigid walls, in which the gas treatment is performed. The cabinet 20 generally comprises a bottom wall 21 and side walls 22, and on its upper side it has an opening through which the head of a patient projects from the cabinet 20.

The cabinet 20 has at least one openable panel that is preferably formed by at least one door 23 on one side, for example on the front side, of the cabinet 20, but the cabinet 20 may also comprise a hinged or removable lid 24, preferably on its upper side. The lid 24, when turned down or being put in place, is adapted for preventing the patient participating in the treatment from bending his or her head into the cabinet 20, and also the treatment gas from escaping from the inner space of the cabinet 20 to a substantial extent.

Inside the cabinet 20 there is a seat 25 on which the patient can sit during the treatment. In the backrest of the seat 25, at least one sensor 26 may be integrated that is capable of indicating the displacement of the patient staying within the cabinet 20 from a secure sitting position (in predetermined directions to a predetermined extent). The sensor 26 may, for example, be a proximity sensor or a pressure sensor. The cabinet 20 may further comprise a sensor 27 for indicating opening or removal of the lid 24, as well as a further sensor 28 for indicating opening of the door 23. The cabinet 20 preferably comprises at least one piece of at least one type of the aforementioned sensors 26, 27 and 28, and more preferably it comprises at least one piece of at least two types of said sensors, and most preferably it comprises at least one piece of all the three types of said sensors.

The cabinet 20 further comprises at least one gas concentration measurement unit 29a for measuring the concentration of the treatment gas adjacent to the upper side of the cabinet 20 during the treatment. The amount of gas fed into the cabinet 20 is regulated on the basis of the signal of the gas concentration measurement unit 29a at a predetermined level or within a predetermined range. In case of a treatment using carbon dioxide gas, the concentration of carbon dioxide is maintained at a level of at most 80% by volume, for example, so that the concentration of the carbon dioxide gas is permanently maintained within a range between 70% and 80% per volume.

The cabinet 20 also has a gas inlet 30 for feeding the treatment gas into the cabinet 20 and an outlet 31 for discharging the gas therefrom. In a preferred embodiment of the apparatus 10, as shown in Figure 1, in addition to the gas concentration measurement unit 29a, another gas concentration measurement unit 29b is also provided within the cabinet 20, preferably at the level of the knees of the patient sitting in the cabinet 20.

The cabinet 20 may further be provided with a handle 32 and also with an adjustable foot rest 33 for enhancing convenience of the patient. The handle 32 is adapted for providing a support for the patient at entering or quitting the cabinet 20 and also while taking his or her position therein. The adjustable foot rest 33 is adapted for supporting the feet of the patient sitting on the seat 25 in a comfortable posture.

To the gas inlet 30 of the cabinet 20, a gas supply unit 40 is coupled through a pipe 41. The gas supply unit 40 controls the amount of gas flowing into the cabinet 20 within a unit of time.

The apparatus 10 further comprises a gas tank 50 coupled to the gas supply unit 40 through a pipe 51. The gas tank 50 is used for storing the treatment gas.

The apparatus 10 further comprises a ventilation unit 60 that is coupled to the outlet 31 through a pipe 61 and to a space 70 hermetically separated from the room containing the cabinet 20, through a pipe 62. The ventilation unit 60 is adapted for an active and high-speed discharge of the gas from the cabinet 20 into the space 70. The space 70 may, for example, be an outdoor space or a suctioning space arranged within the same building.

The apparatus further comprises a control unit 80 that receives the signals of the one or more sensors or measurement units of the group including at least the gas concentration measurement unit 29a, and preferably the sensors 26, 27, 28 and the gas concentration measurement unit 29b, and based on their signals, it controls the operation of the gas supply unit 50 and the ventilation unit 60 according to a prestored control program.

The gas supply unit 40 of the apparatus 10 is preferably provided with a first controllable valve 42 which is preferably formed by a remote-controlled magnetic safety valve. The first valve 42 opens or closes according to the commands of the control unit 80. In its closed state the valve 42 prevents the gas from flowing into the cabinet 20, whereas in its open state the valve 42 allows the gas to flow from the tank 50 into the cabinet 20 through the gas inlet 30.

Additionally, the outlet 31 of the apparatus 10 according to the invention is also provided with a second controllable valve 63, which is preferably arranged in the outlet 31 or adjacent to it, within the pipe 61, and which is preferably formed by a controllable magnetic safety valve or a butterfly valve mounted in the pipe. The second valve 63 opens or closes according to the commands of the control unit 80. In its closed state the valve 63 prevents the gas from flowing out from the cabinet 20, whereas in its open state the valve 63 allows the gas to be pumped from the cabinet 20 by means of the ventilation unit 60.

As it can be seen in Figure 1, the apparatus 10 preferably comprises at least one emergency stop switch 90a, 90b, by means of which the treatment can be immediately stopped at any time. The at least one emergency stop switch 90a, 90b is also in communication with the control unit 80. In a particularly preferred embodiment of the apparatus 10, the apparatus 10 comprises three emergency stop switches, two switches 90a of which being arranged in the inner space of the cabinet 20, for example on the right side and on the left side, with an additional switch 90b being arranged outside the cabinet 20. The inner emergency stop switch 90a within the cabinet 20 can be used by the patient, whereas the outer emergency stop switch 90b can be used by the operating staff to stop the treatment.

Preferably, the apparatus 10 further comprises a detector 100 for measuring the gas concentration around the cabinet 20, said detector being adapted for detecting a specific concentration of the treatment gas in the treatment room containing the cabinet 20 or to analyze the content of the gas mixture in the treatment room. The detector 100 measures the concentration of the one or more gases and optionally, it also analyzes them at a level of approximately 5 to 10 cm in the treatment room. The detector 100 is provided with a built-in power supply, so it can permanently operate even at a power cut. The level of gas concentration to be detected by the detector 100 can be preset. Upon reaching the preset level or range, the detector 100 sends a signal to the control unit 80 which, in turn, controls the operation of the apparatus10 according to this signal.

The connection of the above mentioned sensors, detectors and switches to the control unit 80 of the apparatus 10 according to the invention as well as their communication with the control unit 80 are well known for those skilled in the art, therefore their detailed description is omitted here. In case of gas leakage, the control unit 80 preferably generates a sound signal and it alerts, and it inhibits restarting the treatment while the gas concentration in the treatment room is at a dangerous level.

The normal operation mode and the use of a preferred embodiment of the apparatus 10 shown in Figure 1 will be described below. Before the treatment the door 23 of the apparatus 10 is open and its lid 24 is in a turned-up position or removed, thus the patient to be treated can take his or her place on the seat 25 arranged in the cabinet 20. Once the patient has taken an appropriate posture inside the cabinet 20, the door 23 shall be closed and the lid 24 shall be turned down or put into place, which actions are checked by the sensor 28 indicating opening of the door 23 and the sensor 27 indicating opening or removal of the lid 24, respectively. The treatment can be commenced only if the sensors 27, 28 output a closed-state signal to the control unit 80, i.e. when the door 23 is closed and the lid 24 is turned down or put on the cabinet. Additionally, before the treatment the one or more sensors 26 built in the back rest of the seat 25 or in the inner side of the cabinet also check whether the patient to be treated is in a safe posture for starting the treatment. In a particularly preferred embodiment of the apparatus, both on the right and the left side of the inner space of the cabinet 20, there is a sensor 26 for the sake of even higher safety.

The operation of the apparatus according to the invention shown in Figure 1 will now be described. After starting the treatment the detector 100 measuring the gas concentration of the room containing the cabinet 20 checks whether the level of gas concentration around the cabinet 20 falls within an appropriate range. The control unit 80 starts the treatment only if all of the afore-mentioned safety conditions are complied with, i.e. each of the sensors 26, 27, 28 and 100 sends an enabling signal.

After starting the treatment the gas supply unit 40 directs the gas from the gas tank 50 through the inlet 30 into the cabinet 20 in a controlled way. The inner space of the cabinet 20 is shaped so that during the treatment preferably more than 25% of the body surface of the patient gets into contact with the treatment gas. During the period of the treatment the gas in the cabinet 20 exerts its therapeutic effect in a transcutaneous way.

As the cabinet 20 of the apparatus 10 is not hermetically sealed during the treatment, the concentrations of the gases in the cabinet 20 are continuously measured at least by the gas concentration measurement unit 29a and optionally, also by the gas concentration measurement unit 29b while the cabinet 20 is being filled with the gas and also during the entire period of the treatment. When the level of a gas concentration exceeds a preset threshold associated with a specific measurement height, the control unit 80 switches off the gas supply and closes the first valve 42. When the amount of gas in the cabinet 20 decreases below a preset level, the control unit 80 opens the valve 42 and allows the gas to flow into the cabinet until a preset gas concentration level is reached.

At the end of the treatment the ventilation unit 60 conveys the gas from the cabinet 20 through the outlet 31 into a space 70 which is hermetically separated from the treatment room, for example into the open air. At the end of the treatment the secure removal of the gaseous materials from the cabinet 20 takes at most 5 seconds in a normal operation mode. In case of a normal termination, however, it is recommended to replace the air of the treatment room and to refresh the air therefore the period of gas discharge is preferably set to a longer period, for example 60 seconds. The period of gas feeding and the period of gas discharge can be preset to a time period optimized with respect to the treatment.

During the treatment various hazardous situations can take place with respect to security. These situations may be dangerous both for the patient and the staff operating the apparatus. In such emergency cases, like an excessive gas leakage from the cabinet 20 or the displacement of the patient in a specific direction to a specific extent, opening of any of the openable panels, such as the door 23 or the lid 24, the treatment has to be immediately interrupted. In an emergency case the control unit 80 immediately stops the treatment and removes the gas from the cabinet 20 within the shortest possible time. The emergency stop process commences when at least one of the following events takes place:
- the patient is not sitting in an appropriate posture on the seat 25 (detected by the sensor 26),
- the door 23 of the cabinet 20 has been opened (detected by the sensor 28),
- the lid 24 has been removed (detected by the sensor 27, preferably for a displacement of approximately 1 to 2 cm),
- the gas concentration around the apparatus 10 exceeds a preset gas concentration level (detected by the detector 100),
- any one of the emergency stop switches 90 outputs a signal.

At this time, because of the emergency, one or more of the sensors 26, 27, 28, 100 of the apparatus transit into a disabling state and send an inhibition signal to the control unit 80. Preferably, in their active (disabling) state the sensors 26, 27, 28, 100 also provide an audio or visual alert. The audio alert may, for example, include a sharp, discontinuous sound. The visual alert may, for example, include a text presented on a display. The alert lasts a specific time period, for example about 3 seconds. After the alert has begun, it isn't allowed to re-start the treatment, as the control unit 80 disables the gas supply unit in a programmed manner, and the treatment can be re-started or continued only after an appropriate intervention of an operator.

If after the expiry of an alert period the emergency situation still exists and any one of the sensors 26, 27, 28, 100 is still in a disabling state, the emergency stop process will be initiated.

After initiating the emergency stop process the concentration of the gas in the cabinet 20 has to be reduced within the shortest possible time to a secure concentration level prescribed for the cabinet 20, then the entire amount of gas has to be discharged. The secure gas concentration level of the cabinet 20 is a level at which the concentrations of the gas components right around the patient are below a prescribed secure threshold value. The gas is conveyed by the ventilation unit 60 from the cabinet 20 through the outlet 31 to the space 70 via the pipe 62. During the treatment, in case of an immediate stop at any moment, in order to reach a secure gas concentration level, removal of the gaseous therapeutic materials should preferably be completed in less than 3 seconds, and at least 90% of the gaseous materials is removed within at most 5 seconds. The ventilation unit is a pump unit 60. For example, the pump unit 60 having a power of 350 liters/m³ can withdraw the entire amount of gas from a cabinet 20 having a volume of 350 liters in approximately 3,6 seconds, which is a substantially short period for the patient or any person around to stay in the treatment room without significant health damage even in relatively extreme situations.

After the gas has been entirely withdrawn, the door 23 and the lid 24 of the cabinet 20 can be opened and the patient can quit the apparatus 10 under secure circumstances since in the respiration space of the patient and around the cabinet 20 the concentrations of the gas components are below the secure threshold value. For example, in case of the use of carbon dioxide gas, the threshold value is 0,5% per volume according to the respective health care regulations. Any external intervention or re-start of the treatment is allowed only after the ventilation of the cabinet 20, which is indicated by the end of the alert. After an emergency stop, it is preferred to replace the air of the treatment room and to perform an air refreshment.

As a summary, during its operation the apparatus 10 according to the present invention first conveys the treatment gas into the cabinet in a controlled way, then it holds the gas or the gas mixture within the treatment space in a secure way during the entire period of the treatment, and finally, at the end of the treatment it provides a scheduled discharge of the gas. Furthermore, in any situation regarded as a dangerous event from a point of view of security, it allows a fast removal of the gaseous materials from the inner space of the cabinet in a way that is not dangerous to the patient or the operating staff around the cabinet while being in accordance with the safety regulations.

In the following, the operation of the computer program controlling the security functions of the apparatus according to the present invention will be described.
a) initialization, system boot (enabling module A, without a time limit, recommended period: 120 sec)
b) security check of the inner unit (enabling module B; test of temperature, gas sensors, locks, valves)
c) external check (enabling module K; outer gas sensors, temperature, alert)
d) start of treatment (carried out by the control module I if all of the modules A, B and C are in an enabling state); then the module I fills in the gas mixture up to the level corresponding to the inner gas concentration measurement units (e.g. 29a, 29b)
e) timing (module T, adjusting the period of treatment based on the program selection)
f) normal stop (secure discharge of the gaseous materials (t<5 sec) and conveying them away)
bv.) inner system emergency stop, which stops the modules BV and T, and immediately activates the operation of the termination unit EX.
kv.) external system emergency stop, which stops the modules BV and T, and immediately activates the termination unit EX.

In the above, the module BV is a module for controlling the gas feeding.

In order to reach a secure level, the module EX performs the removal of the gaseous materials preferably within a period t<3 sec, whereas it removes the entire volume of the gaseous materials in a secure way preferably within a period t<5 sec.

## Claims

1. An apparatus (10) for transcutaneous treatment by gas, said apparatus (10) comprising
a rigid-wall cabinet (20) arranged in a treatment room and having an upper opening and at least one openable panel, wherein a seat (25) is arranged inside the cabinet (20), and the cabinet (20) comprises at least one gas concentration measurement unit (29a), and the cabinet (20) further comprises a gas inlet (30) for feeding the treatment gas and a gas outlet (31) for discharging the treatment gas,
a gas supply unit (40) coupled to the gas inlet (30) of the cabinet (20) via a pipe (41) and through an interposed controllable valve (42), and
a gas tank (50) connected to the gas supply unit (40),
a ventilation unit (60) connected to the gas outlet (31) of the cabinet (20) through an interposed controllable valve (63), a control unit (80) for receiving the signals of the gas concentration measurement unit (29a) and for controlling operation of the valves (42, 63) and said ventilation unit (60) based on at least the signals of the gas concentration measurement unit (29a),
wherein the apparatus further comprises at least one emergency sensor (26, 27, 28, 100), and the control unit (80) is further configured to determine an emergency based on the signal of said at least one emergency sensor (26, 27, 28, 100);
**characterized in that**
the cabinet (20) is not hermetically sealed,
said ventilation unit (60) is coupled via a further pipe (62) to a space (70) hermetically separated from the treatment room containing the cabinet (20),
wherein said ventilation unit (60) is a pump unit having a power suitable for removing, in case of emergency determined by the control unit (80), at least 90% of the volume of the treatment gas from the inner space of the cabinet (20) to said hermetically separated space (70) within at most 5 seconds.

2. The apparatus (10) according to claim 1, **characterized in that** the cabinet (20) further comprises at least one sensor for indicating opening of an openable panel, said sensor being coupled to the control unit (80).

3. The apparatus (10) according to claim 1 or 2, **characterized in that** the cabinet (20) further comprises a door (23) as an openable panel on its side, preferably on its front side, wherein the at least one opening indication sensor is a sensor (28) adapted to detect opening of the door (23).

4. The apparatus (10) according to claim 3, **characterized in that** the apparatus further comprises an openable or removable lid (24) on the upper side of the cabinet (20) as an additional openable panel, and a further sensor (27) for indicating displacement of the lid (24).

5. The apparatus (10) according to any one of claims 1 to 4, **characterized in that** the apparatus further comprises a displacement sensor (26) built in a back rest of the seat (25) or in an inner side wall of the cabinet (20), said displacement sensor (26) being in the form of a proximity sensor or a pressure sensor.

6. The apparatus (10) according to any one of claims 1 to 5, **characterized in that** the apparatus further comprises a sensor (100) for measuring the gas concentration around the cabinet (20) and for indicating that the surrounding gas concentration reaches a preset concentration, said sensor (100) being coupled to the control unit (80).

7. The apparatus (10) according to any one of claims 1 to 6, **characterized in that** the apparatus further comprises at least one emergency stop switch (90) coupled to the control unit (80).

## Patentansprüche

1. Einrichtung (10) zur transkutanen Behandlung durch Gas, wobei die Einrichtung (10) umfasst
eine Kabine (20) mit starrer Wand, die in einem Behandlungsraum angeordnet ist und eine obere Öffnung und mindestens eine zu öffnende Platte aufweist, wobei ein Sitz (25) innerhalb der Kabine (20) angeordnet ist, und die Kabine (20) mindestens eine Gaskonzentrationsmesseinheit (29a) umfasst, und die Kabine (20) weiter einen Gaseinlass (30) zum Zuführen des Behandlungsgases und einen Gasauslass (31) zum Abführen des Behandlungsgases umfasst,
eine Gasversorgungseinheit (40), die über ein Rohr (41) und über ein zwischengeschaltetes steuerbares Ventil (42) mit dem Gaseinlass (30) der Kabine (20) gekoppelt ist, und
einen mit der Gasversorgungseinheit (40) verbundenen Gastank (50),
eine Belüftungseinheit (60), die über ein zwischengeschaltetes steuerbares Ventil (63) mit dem Gasauslass (31) der Kabine (20) verbunden ist, eine Steuereinheit (80) zum Empfangen der Signale der Gaskonzentrationsmesseinheit (29a) und zum Steuern eines Betriebs der Ventile (42, 63) und der Belüftungseinheit (60), basierend auf zumindest den Signalen der Gaskonzentrationsmesseinheit (29a),
wobei die Einrichtung weiter mindestens einen Notfallsensor (26, 27, 28, 100) umfasst und die Steuereinheit (80) weiter dazu konfiguriert ist, einen Notfall basierend auf dem Signal des mindestens einen Notfallsensors (26, 27, 28, 100) zu bestimmen;
**dadurch gekennzeichnet, dass**
die Kabine (20) nicht hermetisch verschlossen ist,
die Belüftungseinheit (60) über ein weiteres Rohr (62) mit einem Raum (70) verbunden ist, der hermetisch von dem Behandlungsraum getrennt ist, der die Kabine (20) enthält,
wobei die Belüftungseinheit (60) eine Pumpeneinheit ist, die eine Leistung aufweist, die geeignet ist, in einem Notfall, der von der Steuereinheit (80) bestimmt wird, mindestens 90 % des Volumens des Behandlungsgases aus dem Innenraum der Kabine (20) in den hermetisch getrennten Raum (70) innerhalb von höchstens 5 Sekunden zu entfernen.

2. Einrichtung (10) nach Anspruch 1, **dadurch gekennzeichnet, dass** die Kabine (20) weiter mindestens einen Sensor zum Anzeigen eines Öffnens einer zu öffnenden Platte umfasst, wobei der Sensor mit der Steuereinheit (80) gekoppelt ist.

3. Einrichtung (10) nach Anspruch 1 oder 2, **dadurch gekennzeichnet, dass** die Kabine (20) weiter an ihrer Seite, bevorzugt an ihrer Vorderseite, eine Tür (23) als zu öffnende Platte umfasst, wobei der mindestens eine Öffnungsanzeigesensor ein Sensor (28) ist, der Öffnen der Tür (23) erkennen kann.

4. Einrichtung (10) nach Anspruch 3, **dadurch gekennzeichnet, dass** die Einrichtung weiter einen zu öffnenden oder abnehmbaren Deckel (24) an der Oberseite der Kabine (20) als zusätzliche zu öffnende Platte und einen weiteren Sensor (27) zur Anzeige einer Verschiebung des Deckels (24) umfasst.

5. Einrichtung (10) nach einem der Ansprüche 1 bis 4, **dadurch gekennzeichnet, dass** die Einrichtung weiter einen Verschiebungssensor (26) umfasst, der in eine Rückenlehne des Sitzes (25) oder in eine innere Seitenwand der Kabine (20) eingebaut ist, wobei der Verschiebungssensor (26) in Form eines Näherungssensors oder eines Drucksensors vorliegt.

6. Einrichtung (10) nach einem der Ansprüche 1 bis 5, **dadurch gekennzeichnet, dass** die Einrichtung weiter einen Sensor (100) zum Messen der Gaskonzentration um die Kabine (20) herum und zum Anzeigen, dass die Umgebungsgaskonzentration eine voreingestellte Konzentration erreicht hat, umfasst, wobei der Sensor (100) mit der Steuereinheit (80) gekoppelt ist.

7. Einrichtung (10) nach einem der Ansprüche 1 bis 6, **dadurch gekennzeichnet, dass** die Einrichtung weiter mindestens einen Not-Aus-Schalter (90) umfasst, der mit der Steuereinheit (80) gekoppelt ist.

## Revendications

1. Appareil (10) de traitement transcutané par gaz, ledit appareil (10) comprenant
une enceinte à paroi rigide (20) agencée dans une salle de traitement et présentant une ouverture supérieure et au moins un panneau ouvrable, dans lequel un siège (25) est agencé à l'intérieur de l'enceinte (20), et l'enceinte (20) comprend au moins une unité de mesure de concentration de gaz (29a), et l'enceinte (20) comprend en outre une entrée de gaz (30) pour alimenter le gaz de traitement et une sortie de gaz (31) pour évacuer le gaz de traitement,
une unité d'alimentation en gaz (40) couplée à l'entrée de gaz (30) de l'enceinte (20) par l'intermédiaire d'un tuyau (41) et à travers une soupape commandable interposée (42), et
un réservoir de gaz (50) relié à l'unité d'alimentation en gaz (40),
une unité de ventilation (60) reliée à la sortie de gaz (31) de l'enceinte (20) par l'intermédiaire d'une soupape commandable interposée (63), une unité de commande (80) pour recevoir les signaux de l'unité de mesure de concentration de gaz (29a) et pour commander le fonctionnement des soupapes (42, 63) et de ladite unité de ventilation (60) sur la base d'au moins les signaux de l'unité de mesure de concentration de gaz (29a),
dans lequel l'appareil comprend en outre au moins un capteur d'urgence (26, 27, 28, 100), et l'unité de commande (80) est en outre configurée pour déterminer une urgence sur la base du signal dudit au moins un capteur d'urgence (26, 27, 28, 100) ;
**caractérisé en ce que**
l'enceinte (20) n'est pas hermétiquement fermée,
ladite unité de ventilation (60) est couplée par l'intermédiaire d'un autre tuyau (62) à un espace (70) hermétiquement séparé de la salle de traitement contenant l'enceinte (20),
dans lequel ladite unité de ventilation (60) est une unité de pompe présentant une puissance adaptée pour évacuer, en cas d'urgence déterminée par l'unité de commande (80), au moins 90 % du volume du gaz de traitement depuis l'espace intérieur de l'enceinte (20) vers ledit espace hermétiquement séparé (70) en au plus 5 secondes.

2. Appareil (10) selon la revendication 1, **caractérisé en ce que** l'enceinte (20) comprend en outre au moins un capteur pour indiquer l'ouverture d'un panneau ouvrable, ledit capteur étant couplé à l'unité de commande (80).

3. Appareil (10) selon la revendication 1 ou 2, **caractérisé en ce que** l'enceinte (20) comprend en outre une porte (23) en tant que panneau ouvrable sur son côté, de préférence sur son côté avant, dans lequel le au moins un capteur d'indication d'ouverture est un capteur (28) adapté pour détecter l'ouverture de la porte (23).

4. Appareil (10) selon la revendication 3, **caractérisé en ce que** l'appareil comprend en outre un couvercle ouvrable ou amovible (24) sur le côté supérieur de l'enceinte (20) en tant que panneau ouvrable supplémentaire, et un autre capteur (27) pour indiquer le déplacement du couvercle (24).

5. Appareil (10) selon l'une quelconque des revendications 1 à 4, **caractérisé en ce que** l'appareil comprend en outre un capteur de déplacement (26) intégré dans un dossier du siège (25) ou dans une paroi latérale intérieure de l'enceinte (20), ledit capteur de déplacement (26) se présentant sous la forme d'un capteur de proximité ou d'un capteur de pression.

6. Appareil (10) selon l'une quelconque des revendications 1 à 5, **caractérisé en ce que** l'appareil comprend en outre un capteur (100) pour mesurer la concentration de gaz autour de l'enceinte (20) et pour indiquer que la concentration de gaz environnant atteint une concentration prédéfinie, ledit capteur (100) étant couplé à l'unité de commande (80).

7. Appareil (10) selon l'une quelconque des revendications 1 à 6, **caractérisé en ce que** l'appareil comprend en outre au moins un interrupteur d'arrêt d'urgence (90) couplé à l'unité de commande (80).
